# EUROPÄISCHE PATENTSCHRIFT

(11) **EP 2 575 662 B1**
(45) Veröffentlichungstag und Bekanntmachung des Hinweises auf die Patenterteilung: **18.10.2017**
(21) Anmeldenummer: 11717552.1
(22) Anmeldetag: 21.04.2011
(51) Int. Cl.: A61B 34/00

(54) **VERFAHREN ZUM BEWEGEN EINES INSTRUMENTENARMS EINES LAPAROSKOPIEROBOTERS IN EINE VORGEBBARE RELATIVLAGE ZU EINEM TROKAR**
METHOD FOR MOVING AN INSTRUMENT ARM OF A LAPAROSCOPY ROBOT INTO A PREDETERMINABLE RELATIVE POSITION WITH RESPECT TO A TROCAR
PROCÉDÉ DE DÉPLACEMENT DU BRAS PORTE-INSTRUMENTS D'UN ROBOT DE LAPAROSCOPIE DANS UNE POSITION RELATIVE PRÉDÉFINISSABLE PAR RAPPORT À UN TROCART

(30) Priorität: 25.05.2010 DE 102010029275
(43) Veröffentlichungstag der Anmeldung: 10.04.2013
(73) Patentinhaber: Siemens Healthcare GmbH, 91052 Erlangen (DE)
(72) Erfinder: BÄRWINKEL, Ronny, 91077 Dormitz (DE); HORNUNG, Oliver, 90768 Fürth (DE); MAIER, Karl-Heinz, 90518 Altdorf b. Nürnberg (DE)
(86) Internationale Anmeldenummer: PCT/EP2011/056438
(87) Internationale Veröffentlichungsnummer: WO 2011/147651

(56) Entgegenhaltungen:
- EP-A1- 1 854 425
- WO-A2-01/46577
- US-A1- 2006 258 938
- US-A1- 2009 088 897
- US-A1- 2010 081 875
- DOIGNON C ET AL: "Autonomous 3-d positioning of surgical instruments in robotized laparoscopic surgery using visual servoing", IEEE TRANSACTIONS ON ROBOTICS AND AUTOMATION, IEEE INC, NEW YORK, US, Bd. 19, Nr. 5, 1. Oktober 2003 (2003-10-01), Seiten 842-853, XP011102058, ISSN: 1042-296X, DOI: DOI:10.1109/TRA.2003.817086

## Beschreibung

Die Erfindung betrifft ein Verfahren zum Bewegen eines Instrumentenarms eines Laparoskopieroboters in eine vorgebbare Relativlage relativ zu einem in einem Patienten platzierten Trokar.

Minimalinvasive Eingriffe nehmen im Bereich der klinischen Chirurgie einen zunehmend größeren Stellenwert ein. Wurden für kleine chirurgische Eingriffe noch vor wenigen Jahren relativ große Bereiche des Situs eröffnet, um eine Navigation des Chirurgen durch natürliche Landmarken zu ermöglichen, so lässt sich beobachten, dass eine Vielzahl dieser Eingriffe heute mittels Laparoskopie und optischer Unterstützung in Form von Endoskopie durchgeführt wird. Als Weiterentwicklung der klassischen Laparoskopie hat die robotergestützte Chirurgie inzwischen in einigen Bereichen der Medizin Einzug gehalten, z.B. in der Urologie, Gynäkologie oder Kardiologie. Sie ist dabei, sich im medizinischen Alltag durchzusetzen.

Aus EP 1 854 425 A1 ist beispielsweise ein chirurgischer Roboter bekannt, dessen Instrumentenarm anhand von Positionsinformationen steuerbar ist.

Ein Laparoskopieroboter ist zum Beispiel in Form des Modells "da Vinci" der Firma "Intuitive Surgical" bekannt. Dieser Roboter weist einen ersten Instrumentenarm auf, der an seinem vorderen Ende ein Endoskop trägt. Bis zu drei weitere Instrumentenarme tragen laparoskopische Instrumente.

Sowohl für das Endoskop als auch jedes der Instrumente wird im Patienten jeweils ein Trokar platziert, durch welchen das jeweilige Werkzeug in den Patienten einzuführen ist, um sodann einen roboterassistierter, minimalinvasiven Eingriff am Patienten durchzuführen.

Im klinischen Workflow eines derartigen Eingriffs lässt sich beobachten, dass ein wesentlicher Anteil der Operationsvorbereitungszeit darin besteht, die vom Roboter an den Instrumentenarmen geführten Werkzeuge in die bereits im Vorfeld der Operation gesetzten Trokare einzuführen bzw. dieses Einführen vorzubereiten. Am jeweiligen Roboterarm werden hierzu dessen Einzelgelenke, Linearaktoren etc. kraftfrei geschaltet und der Instrumentenarm so von Hand eingestellt, dass durch die entsprechenden Gelenkstellungen beziehungsweise der zu Grunde liegenden Kinematik des Roboters das Instrument - noch außerhalb des Patienten - in einer vorgegebenen Relativlage, die auch als Ziellage verstanden werden kann, zum Trokar zu liegen kommt. Der Trokar ist hierbei in einer individuellen Ortslage bereits im Patienten - mehr oder weniger ortsfest - gesetzt. Ein Trokar gibt hierbei eine Trokarachse vor, in der Regel dessen Mittellängsachse als Richtung, in der die länglichen Werkzeuge axial durch den Trokar in den Patienten eingeführt werden.

Beim da Vinci-System ist der Instrumentenarm zusammen mit dem Instrument so - in der Regel in drei Dimensionen - auszurichten, dass die Längsachse des gehaltenen Instruments koaxial zur Trokarachse verläuft, sich das Instrument jedoch noch zum Trokar beabstandet und außerhalb des Patienten liegt. Mit anderen Worten ist die vorgegebene Relativlage so, dass das Instrument am Instrumentenarm den Trokar mit einer geradlinigen Bewegung treffen bzw. durchdringen kann. Das Instrument ist dann noch in dieser axialen Richtung eindimensional bis zur Berührung an den Trokar anzufahren. Der Instrumentenarm wird dann wieder kraftgekoppelt; robotisch erfolgt dann die geradlinige Einführung der Instrumente in den jeweiligen Trokar. Mit anderen Worten existiert also eine anzusteuernde vorgebbare Relativlage für den Instrumentenarm, die relativ zu einem in einem Patienten platzierten Trokar vorgebbar ist.

Dieser Vorgang der manuellen Justage setzt einige Erfahrung des vorbereitenden Personals voraus und ist sehr zeitaufwändig. Die händische Verstellung ist selbst bei mechanisch ausgefeilten Systemen sehr kraftaufwendig, da die Reibung in den Gelenken aus Sicherheitsgründen auch bei kraftlosem Betrieb sehr hoch gewählt werden muss. Im Fall des oben genannten da Vinci-Systems muss dieses Prozedere bei Verwendung aller Roboterarme viermal wiederholt werden.

Aufgabe der vorliegenden Erfindung ist es, ein verbessertes Verfahren zum Bewegen eines Instrumentenarms eines Laparoskopieroboters in eine vorgebbare Relativlage relativ zu einem in einem Patienten platzierten Trokar anzugeben.

Die Aufgabe wird gelöst durch ein Verfahren gemäß Patentanspruch 1. Gemäß der Erfindung wird am Trokar ein von außerhalb des Patienten ortbarer Ortsmarker angebracht. Die Ortsposition des Trokars wird anhand des Ortsmarkers erfasst. Zum Beispiel wird also von einem Navigationssystem die Raumlage, also Position und Orientierung des Trokars in z.B. einem im Operationssaal ortsfesten Koordinatensystem anhand des Ortsmarkers erfasst. Aus der Ortsposition und der vorgegebenen Relativlage des Instrumentenarms zum Trokar wird dann die Sollposition des Instrumentenarms im Raum ermittelt.

In einem nächsten Schritt wird die Ist-Position des Instrumentenarms im Raum erfasst. Hierzu kann zum Beispiel auch am Instrumentenarm ein ortbarer Ortsmarker angebracht werden, um so die Raumlage als Ist-Position des Instrumentenarms zu erfassen. Die Raumlage kann aber auch anhand von Positionsaufnehmern im Roboterarm bei bekannter Position eines Grundträgers des Roboters oder anhand anderer Methoden erfasst werden.

Anhand der Ist-Position und der Soll-Position des Instrumentenarms wird dann mit Hilfe eines Fehlerminimierungsverfahrens der Instrumentenarm im Rahmen einer Operationsvorbereitung in die Soll-Position bewegt. Mit anderen Worten wird die ständige Ist-Position des Instrumentenarms stets weiterverfolgt d.h. während der außerhalb des Patienten ablaufenden Bewegung wiederholt erfasst und anhand des Fehlerminimierungsverfahrens erreicht, dass sich die Ist-Position immer mehr der Soll-Position annähert und schließlich in diese überführt wird. In der Soll-Position liegt dann der Instrumentenarm in der vorgebbaren Relativlage relativ zum Trokar. Grundlage zur Ortung bilden zum Beispiel mindestens drei statisch auf dem Trokar auf der Außenseite des Körpers des Patienten angeordnete bzw. mit diesem verbundene Marker. Diese werden dann z.B. von einer CCD-Kamera in deren Kamerabild erfasst und verfolgt. Vorteilhaft ist die Auswahl von im Kamerabild sehr gut segmentierbaren Markern. Zum Beispiel können Infrarot(IR)-reflektierende Marker und eine IR-Lichtquelle aus Richtung der Kamera verwendet werden. Eine solche Vorrichtung ist zum Beispiel aus Trackinggeräten wie dem System "Polaris" der Firma NDI bekannt.

Das erfindungsgemäße Verfahren eignet sich sowohl für einen einzelnen Instrumentenarm eines mehrarmigen Roboters wie auch für den oben genannten einarmigen Single-Port-Roboter. Der Gewinn an wertvoller Operationszeit, eine kürzere Narkosezeit und eine einfachere Bedienung des Gerätes sind die direkten Folgen des erfindungsgemäßen Verfahrens.

Die Bewegung des Roboterarms gemäß dem Verfahren kann hierbei weiterhin von Hand erfolgen. Zum Beispiel werden dem Bediener jedoch als Ergebnis der verfahrensgemäßen Fehlerminimierung durchzuführende Verstellbewegungen für einzelne Gelenke oder Freiheitsgrade des Roboters vorgeschlagen. So kann auch die händische Bewegung des Instrumentenarms zielgerichtet bzw. ergebnisorientiert und daher deutlich verbessert durchgeführt beziehungsweise beschleunigt werden. Das zuständige OP-Personal muss nun nicht mehr "nach Gefühl oder Augenmaß" den Roboterarm verstellen, sondern erhält konkrete Hilfestellung dank des erfindungsgemäßen Verfahrens durch konkret angewiesene Verstellvorschläge.

In einer vorteilhaften Ausführungsform des Verfahrens jedoch wird der Instrumentenarm automatisch in die Soll-Position bewegt. Somit erfolgt eine vollständig automatisierte, also vom Roboter selbst motorisch vollzogene Ausrichtung des Instrumentenarms zum Trokar. Anschließend kann dann sofort mit der eigentlichen Behandlung des Patienten beziehungsweise deren erstem Schritt, nämlich dem robotergestützten Einführen der Instrumente in den Patienten begonnen werden.

Die Relativlage kann noch einen Freiheitsgrad enthalten: Ist zum Beispiel die Relativlage so vorgegeben, dass das Instrument koaxial zur Durchtrittsachse des Trokars liegt, reduziert sich eine verbleibende händische Operationsvorbereitung für das Personal von einem dreidimensionalen zu einem eindimensionalen Problem: Das Instrument muss lediglich noch entlang einer Linie, nämlich der Mittellängsachse des Trokars zum Beispiel axial bis zum Trokar vorgeschoben werden, um den Roboter endgültig am Patienten zu justieren. Dieser händische Schritt ist z.B. der, das Instrument auf Berührung am Trokar zu platzieren und so eine Nulllage im System zu definieren.

In einer bevorzugten Ausführungsform des Verfahrens wird als Relativlage eine solche gewählt, die eine koaxiale Ausrichtung eines axial in den Trokar einführbaren, am Instrumentenarm angebrachten Instruments gewährt. Es erfolgt genauer gesagt eine koaxiale Ausrichtung einer Instrumentenachse als z.B. Mittellängsachse eines Instruments mit einer Trokarachse als z.B. Mittellängsachse des Trokars. Die Position des Instruments wird also als Relativlage bzw. Ziellage definiert. Dies eignet sich besonders für Roboter, bei denen das Instrument selbst in axialer Richtung - z.B. zur händischen Erreichung einer berührenden Nullage am Trokar - verstellbar ist. Als Relativlage kann dann die o.g. Lage außerhalb des Patienten und mit einem Sicherheitsabstand zum Trokar gewählt werden.

In einer weiteren vorteilhaften Ausführungsform wird die Ortsposition des Trokars und/oder die Ist- und Soll-Position des Instrumentenarms durch eine optische Erfassung mittels einer Kamera ermittelt. Mit anderen Worten wird zur Ortung dann ein optisch arbeitendes Navigationssystem benutzt. Die optische Erfassung geschieht zum Beispiel an Hand einer Kamera, die Ortsmarker sind dann optisch sichtbar. Denkbar sind als Marker zum Beispiel mindestens drei am Trokar angebrachte Markerpunkte, welche durch die Kamera erfassbar sind. Bekannte Kamerasysteme arbeiten mit sichtbarem Licht oder Infrarotlicht.

In einer bevorzugten Ausführungsform des Verfahrens wird die oben genannte optische Erfassung der entsprechenden Positionen bzw. Marker derart gelöst, dass hierzu ein Endoskop, welches an einem der Instrumentenarme eines Roboters angeordnet ist, als Kamera verwendet wird. Mit anderen Worten wird gemäß dieser Ausgestaltung das Endoskop, welches ohnehin Kamerafunktionalität aufweist, als Navigationskamera außerhalb des Patienten mitbenutzt. Ist außerdem das Endoskop an demjenigen Instrumentenarm angeordnet, der zur Relativlage bewegt werden soll, entfällt ein zusätzlicher Schritt bei der Positionsbestimmung des Instrumentenarms: Die Kamera selbst beinhaltet dann die zu ortende Position des Instrumentenarms in sich. Mit anderen Worten entartet die jeweilige Ist-Position bzw. die Soll-Position zur Kameraposition. Die Kamera kann dann auch direkt durch optische Erfassung des Ortsmarkers am Trokar die Relativlage zwischen Trokar und Kamera und damit dem Instrumentenarm ermitteln.

Auch für mehrarmige Roboter entfällt bei Verwendung eines Endoskops an einem Instrumentenarm die Notwendigkeit die Positionen der anderen Arme zusätzlich zu erfassen: Die Position der Instrumentenarme sind hierbei jeweils stets bekannt, da ohnehin das gesamte System aus seiner Natur heraus bezüglich seiner Geometrie kalibriert beziehungsweise ortsregistriert ist.

Wie oben erwähnt, ist in einer bevorzugten Ausführungsform des Verfahrens als Instrumentenarm ein Kombinationsarm für eine Single-Port-Technik verwendet. Insbesondere wenn das Endoskop des Single-Port-Roboters als Kamera verwendet wird, muss nur eine einzige Positionserfassung erfolgen, die bereits die Relativlage zum Trokar wiederspiegelt. Die Geometrien der restlichen Instrumente zum Endoskop sind ohnehin aus der Geometrie ihrer Fixanordnung am Instrumentenarm bekannt. In einer bevorzugten Ausführungsform des Verfahrens wird als Fehlerminimierungsverfahren ein Visual-Servoing-Verfahren durchgeführt. Insbesondere in Verbindung mit der Verwendung des Endoskops als Ortungskamera sind Visual-Servoing-Ansätze besonders zur Ausrichtung zwischen Instrumentenarm und Trokar geeignet. Die Lage der Marker beziehungsweise Bildmerkmale im aktuellen Kamerabild ist bekannt. So können zwei verschiedene Visual-Servoing-Ansätze durchgeführt werden:
Ein erster Ansatz ist das positionsbasierte Visual-Servoing. Aus den Projektionen der Marker im Kamerabild, also der Lage der Bildmerkmale, wird bei bekannter Geometrie zwischen den Markern, z.B. am Trokar und der kalibrierten Kamera die Lage - also Position und Orientierung - des Trokars in Bezug auf das Kamerakoordinatensystem geschätzt bzw. ermittelt. Durch ein geeignetes Regelgesetz als Fehlerminimierungsverfahren können dann das Endoskop oder Instrument bzw. ein Instrumentenarm in die zuvor vorgegebene Relativlage überführt werden.

Als zweiter Ansatz dient ein bildbasiertes Visual-Servoing: Die wie oben gewonnenen Projektionen des Ortsmarkers im Kamerabild werden hier durch ein geeignetes Regelgesetz als Fehlerminimierungsverfahren auf gewünschte festgegebene Bildpositionen geführt. Hier werden also durch Vorgabe der Relativlage feste Positionen der Abbildungen des Ortsmarkers im Kamerabild festgelegt.

Insbesondere für das bildbasierte Visual-Servoing gilt, wenn die Kamera am zu justierenden Instrumentenarm angebracht ist: Sobald sich die Ortsmarker in einer gewünschten Bildlage im Kamerabild befinden, befindet sich automatisch auch der Instrumentenarm in der gewünschten Lage, da die Kamera am Instrumentenarm in einer festen und bekannten Position fixiert angeordnet ist und mit diesem mitbewegt wird.

Prinzipiell sind beide Visual-Servoing-Ansätze bzw. - Verfahren im vorliegenden Fall verwendbar, wobei beide Verfahren Vor- und Nachteile aufweisen. Beim bildbasierten Visual-Servoing überwiegen hier jedoch die Vorteile, weshalb dieses Verfahren zu bevorzugen ist.

In einer bevorzugten Ausführungsform des Verfahrens wird daher ein bildbasiertes Visual-Servoing-Verfahren durchgeführt.

Um bei einem derartigen Verfahren und einer fest am Instrumentenarm angebrachten Kamera die Kamera so zu bewegen, dass die gewünschten Bildpunkte in einer vorher festgelegte Referenzposition wandern, ist es notwendig die Abbildungsvorschrift zwischen einer gewünschten Bildpunktänderung, d.h. der Änderung des Markerabbilds und der korrespondierenden Kameralageänderung herzustellen:
In einer bevorzugten Ausführungsform wird die gesuchte Lageänderung über eine Bild-Jacobi-Matrix als Vorschrift ermittelt. Die Bild-Jacobi-Matrix ist beispielsweise in "A.C. Sanderson, L.E. Weiss and C.P. Neuman, 'Dynamic sensor-based control of robots with visual feedback', IEEE Journal of Robotics and Automation 3(5) (1987), 404-417" bekannt und wird erfindungsgemäß im Fehlerminimierungsverfahren verwendet. Dessen Regelverhalten lässt sich daraufhin über ein proportional-integrales Regelgesetz sehr gut beeinflussen.

Die oben genannte Relativlage kann jeweils abstrakt, z.B. in Form von 3D-Koordinaten und Winkellagen in einem ortsfesten 3D-Korrdinatensystem des OP-Saales vorgegeben werden.

In einer bevorzugten Ausführungsform des Verfahrens wird jedoch die Relativlage durch ein Lernverfahren bzw. einen sogenannten tech-in-Schritt gewonnen. Zum Beispiel wird dabei einmalig ein Instrumentenarm mit fest angebrachter Endoskopkamera händisch in eine gewünschte Relativlage zu einem Trokar gebracht und vom Endoskop ein Zielbild des Trokarmarkers aufgenommen. Die Relativlage gibt hierbei die absolute Ziellage zwingend vor. So ergibt sich im Zielbild eine Soll-Lage der Markerpunkte im Kamerabild, die zu einem späteren Zeitpunkt wieder einzunehmen ist. Die Ziellage der Markerpunkte im Bild ist dann durch den oben erläuterten teach-in-Schritt vorgegeben.

Ein Visual-Servoing-Verfahren ist zum Beispiel aus "J. Wang and W.J. Wilson, '3D relative position and orientation estimation using Kalman filter for robot control', 1992 IEEE Int. Conf. On Robotics and Automation, pp. 2638-2645 (1992)" bekannt.

Für eine weitere Beschreibung der Erfindung wird auf die Ausführungsbeispiele der Zeichnungen verwiesen. Es zeigen, jeweils in einer schematischen Prinzipskizze:
Fig.1 einen Patienten mit Endoskopieroboter,
Fig.2 ein Visual-Servoing-Verfahren mit Bildjacobimatrix.

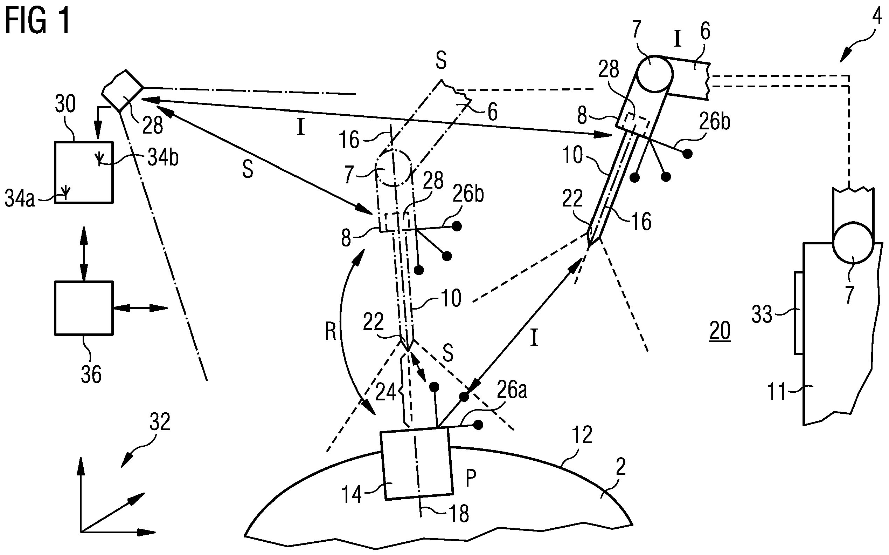

Fig. 1 zeigt einen Patienten 2, an dem ein laparoskopischer Eingriff durchgeführt werden soll. Am Patienten 2 befindet sich daher ein Laparoskopie-Roboter 4, der einen über mehrere Gelenke 7 beweglichen Instrumentenarm 6 aufweist. Der Instrumentenarm 6 trägt an seinem Ende 8 als Instrument 10 eine Greifzange, mit der der Eingriff durchgeführt werden soll.

Um einen Patientenzugang zu schaffen, ist in den Patienten 2, bzw. in eine Öffnung in dessen Bauchdecke 12, ein Trokar 14 eingesetzt. Um in den Patienten 2 zu gelangen, muss das Instrument 10 mit seiner Mittellängsachse, nämlich der Instrumentenachse 16, koaxial zur Mittellängsachse des Trokars 14, also der Trokarachse 18, nämlich dessen nicht dargestellter zentraler Öffnung ausgerichtet werden. Dies sowie die stilisierte Verbindung des Instrumentenarms 6 zu einem Grundträger 11 des Roboters 4 ist in Fig. 1 gestrichelt dargestellt.

Die Ausrichtung soll noch außerhalb des Patienten 2, das heißt im Außenraum 20 erfolgen, wobei die Spitze 22 des Instruments 10 noch einen vorgegebenen Abstand 24 zum Trokar 14 aufweisen soll. Fig. 1 zeigt die entsprechende Position des Instrumentenarms 6 als Soll-Position S und gleichzeitig die tatsächliche Ist-Position I, in welcher er sich gerade befindet. Nach dem erfindungsgemäßen Verfahren soll der Instrumentenarm 6 aus der Ist-Position I in die Soll-Position S überführt werden.

Hierzu ist am Trokar 14 ein Ortsmarker 26a angebracht. Der Ortsmarker 26a wird durch eine Kamera 28 erfasst. Die Kamera 28 bildet den Ortsmarker 26a in einem Kamerabild 30 ab. Auf Grund der Auswertung des Kamerabildes 30 beziehungsweise des Abbildes des Ortsmarkers 26a und der bekannten Abbildungsgeometrie der Kamera 28 sowie der Position der Kamera 28, zum Beispiel in einem raumfesten Koordinatensystem 32, wird die Ortsposition P des Trokars 14 im Raum, also dem Koordinatensystem 32 ermittelt.

In einem nächsten Schritt wird die Ist-Position I des Instrumentenarms 6 ermittelt, wozu am Instrumentenarm 6 ebenfalls ein Ortsmarker 26b angebracht wird, welcher in gleicher Weise von der Kamera 28 erfasst und ausgewertet wird. Dargestellt sind die jeweiligen Abbilder 34a,b der Ortsmarker 26a,b im Kamerabild 30.

Die Soll-Position S für den Instrumentenarm 6 ist nun bestimmt durch die tatsächliche Ortsposition P des Trokars 14 sowie eine vorgebbare Relativlage R, die der Instrumentenarm 6 relativ zum Trokar 14 einnehmen soll.

Da nun im Koordinatensystem 32 sämtliche geometrischen Informationen vorliegen, bestimmt ein schematisch dargestelltes Fehlerminimierungsverfahren 36 anhand des Kamerabildes 30 beziehungsweise der Ist-Position I und der Soll-Position S geeignete Bewegungen für den Instrumentenarm 6 beziehungsweise dessen Gelenke 7, nämlich wie diese zu verstellen sind, um den Instrumentenarm 6 in die Soll-Position S und damit die gewünschte Relativlage R zum Trokar 14 zu bringen.

In einer ersten Ausführungsformen wird, z.B. an einer Anzeige 33 oder in anderer geeigneter Weise, einem nicht dargestellten Bediener angezeigt, wie er jeden Freiheitsgrad des Instrumentenarms, im Beispiel die Gelenke 7, zu verstellen hat, um zur Soll-Position S zu gelangen.

In einer alternativen Ausführungsform werden die Gelenke 7 durch den Roboter 4 an Hand des Fehlerminimierungsverfahrens 36 automatisch bzw. motorisch verstellt.

In einer alternativen Ausführungsform ist der Trokar 14 ein Single-Port-Trokar. Das Instrument 10 besteht dann aus einer Reihe parallel angeordneter, nicht dargestellter Einzelinstrumente, von denen eines auch in der Regel ein Endoskop ist, so dass das im folgenden beschriebene Verfahren zur Erreichung der Soll-Position S verwendet werden kann, da sämtliche Teilinstrumente in fester und bekannter Relativanordnung zueinander auch als Kombinationsinstrument 10 betrachtet werden können.

In einer alternativen Ausführungsform ist das Instrument 10 ein Endoskop, angedeutet durch eine gestrichelt gezeichnete Kamera 28 im Instrumentenarm 6. Der jeweilige Bildbereich bzw. Blickwinkel der Kamera 28 geht von der Spitze 22 des Instruments 10 aus (gestrichelt angedeutet). Die Ortsmarker 26b sind in diesem Fall nicht vorhanden, die Kamera 28 ortet alleine den Ortsmarker 26 am Trokar 14. Da in dieser Ausführungsform die Kamera jeder Bewegung des Instrumentenarms 6 zwangsläufig folgt, ist das Kamerabild 30 und die daraus gewonnene Geometrieinformation unweigerlich mit der tatsächlichen Ist-Position I des Instrumentenarms 6 verknüpft. Die Soll-Position S entspricht dann der Relativlage R.

Hier wird das Fehlerminimierungsverfahren 36 derart durchgeführt, dass im Kamerabild 30 einzig das Abbild 34a des Ortsmarkers 26a an eine fest vorgebbare Soll-Lage zu bringen ist. Diese Soll-Lage des Abbilds 34a gibt dann zwangsläufig sowohl die Soll-Position S als auch die gewünschte Relativlage R vor beziehungsweise stimmt mit dieser überein. Das Fehlerminimierungsverfahren 36 ist in diesem Fall ein bildbasiertes Visual-Servoing-Verfahren.

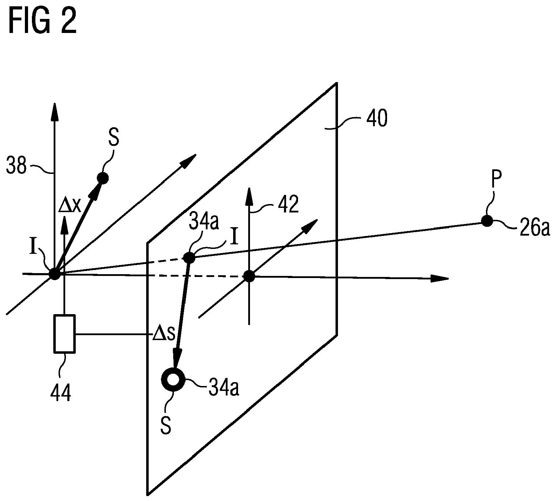

Fig. 2 zeigt zur Verdeutlichung des bildbasierten Visual-Servoing-Verfahrens für die oben genannte endoskopische Verfahrensvariante stellvertretend für die Kamera 28 deren Kamerakoordinatensystem 38 an der Ist-Position I und stellvertretend für das Kamerabild 30 die Abbildungsebene 40 der Kamera 28. Zusätzlich ist die tatsächliche Raumlage, das heißt die Ortsposition P des Ortsmarkers 26a symbolisch dargestellt. In der Abbildungsebene 40 erscheint deshalb dessen Abbild 34a. Die Abbildungsebene 40 spannt ein Bildkoordinatensystem 42 auf.

Durch einen vorherigen teach-in-Schritt, bei welchem sich in einem Kalibrierverfahren der Instrumentenarm 6 bereits an der Soll-Position S, das heißt in gegebener Relativposition R gegenüber dem Ortsmarker 26a befunden hatte, ist die Soll-Position S des Abbildes 34a des Ortsmarkers 26a bekannt. Hierzu wurde einmalig ein Kamerabild 30 als Referenzbild aufgenommen. Gesucht ist nun der Verfahrweg, den das Kamerakoordinatensystem 38 - und damit die Kamera 28 - von der Ist-Position I zur Soll-Position S ausführen müsste, damit das Abbild 34a in der Abbildungsebene 40 von der Ist-Position I in die Soll-Position S wandert. Die Berechnung erfolgt anhand einer Bild-Jacobi-Matrix 44, die die bekannte Verschiebung Δs des Abbildes 34a in die tatsächlich notwendige Verschiebung Δx des Kamerakoordinatensystems 38 und damit der Kamera 28 und damit des Instrumentenarms 6 überführt.

## Patentansprüche

1. Verfahren zum Bewegen eines Instrumentenarms (6) eines Laparoskopieroboters (4) im Rahmen einer Operationsvorbereitung, wobei die Bewegung des Instrumentenarms (6) außerhalb eines Patienten (2) derart erfolgt, dass ein am Instrumentenarm (6) angebrachtes Instrument (10) in eine vorgebbare Relativlage (R) relativ zu einem im Patienten (2) platzierten Trokar (14) positioniert wird, wobei das Instrument (10) in der Relativlage (R) in einem Sicherheitsabstand zum Trokar (14) außerhalb des Patienten (2) angeordnet ist und
- am Trokar (14) ein von außerhalb des Patienten (2) ortbarer Ortsmarker (26a) angebracht wird,
- die Ortsposition (P) des Trokars (14) anhand des Ortsmarkers (26a) erfasst wird,
- die Soll-Position (S) des Instrumentenarms (6) aus der Ortsposition (P) und der Relativlage (R) ermittelt wird,
- die Ist-Position (I) des Instrumentenarms (6) erfasst wird,
- anhand der Ist-Position (I), der Soll-Position (S) und eines Fehlerminimierungsverfahrens (36) der Instrumentenarm (6) in die Soll-Position (S) bewegt wird.

2. Verfahren nach Anspruch 1, bei dem der Instrumentenarm (6) automatisch in die Soll-Position (S) bewegt wird.

3. Verfahren nach einem der vorhergehenden Ansprüche, bei dem als Relativlage (R) eine solche gewählt wird, die eine koaxiale Ausrichtung eines mit seiner Instrumentenachse (16) entlang einer Trokarachse (18) axial in den Trokar (14) einführbaren, am Instrumentenarm (6) angebrachten Instruments (10) gewährt.

4. Verfahren nach einem der vorhergehenden Ansprüche, bei dem die Ortsposition (P) und/oder Ist- (I) und Soll-Position (S) durch eine optische Erfassung durch eine Kamera (28) ermittelt werden.

5. Verfahren nach Anspruch 4, bei dem am Instrumentenarm (6) ein Endoskop als Kamera (28) angeordnet wird.

6. Verfahren nach einem der vorhergehenden Ansprüche, bei dem als Instrumentenarm (6) ein Kombinationsarm für eine Single-Port-Technik verwendet wird.

7. Verfahren nach einem der vorhergehenden Ansprüche, bei dem als Fehlerminierungsverfahren (36) ein Visual-Servoing-Verfahren durchgeführt wird.

8. Verfahren nach Anspruch 7, bei dem als Fehlerminierungsverfahren (36) ein bildbasiertes Visual-Servoing-Verfahren durchgeführt wird.

9. Verfahren nach Anspruch 8, bei dem das Fehlerminierungsverfahren (36) anhand einer Bildjacobimatrix (44) durchgeführt wird.

10. Verfahren nach einem der vorhergehenden Ansprüche, bei dem die Relativlage (R) durch Lernverfahren gewonnen wird.

## Claims

1. Method for moving an instrument arm (6) of a laparoscopy robot (4) in the context of a surgical preparation, wherein the movement of the instrument arm (6) outside the patient (2) takes place such that an instrument (10) attached to the instrument arm (6) is positioned in a predeterminable relative position (R) relative to a trocar (14) placed in the patient (2), wherein the instrument (10) is arranged in the relative position (R) at a safe distance from the trocar (14) outside the patient (2) and
- a spatial marker (26a) that can be located from outside the patient (2) is applied to the trocar (14),
- the spatial position (P) of the trocar (14) is detected on the basis of the spatial marker (26a),
- the desired position (S) of the instrument arm (6) is established from the spatial position (P) and the relative position (R),
- the actual position (I) of the instrument arm (6) is detected,
- the instrument arm (6) is moved into the desired position (S) on the basis of the actual position (I), the desired position (S) and an error minimisation method (36).

2. Method according to claim 1, in which the instrument arm (6) is moved automatically into the desired position (S).

3. Method according to one of the preceding claims, in which the position selected as a relative position (R) is such that it guarantees a coaxial alignment of an instrument (10) attached to the instrument arm (6), which instrument (10) can be inserted axially into the trocar (14) with its instrument axis (16) along a trocar axis (18).

4. Method according to one of the preceding claims, in which the spatial position (P) and/or actual (I) and desired position (S) are determined by optical detection by a camera (28).

5. Method according to claim 4, in which an endoscope is arranged on the instrument arm (6) as a camera (28).

6. Method according to one of the preceding claims, in which a combination arm for a single-port system is used as an instrument arm (6).

7. Method according to one of the preceding claims, in which a visual servoing method is carried out as an error minimisation method (36).

8. Method according to claim 7, in which an image-based visual servoing method is carried out as an error minimisation method (36).

9. Method according to claim 8, in which the error minimisation method (36) is carried out using an image Jacobian matrix (44).

10. Method according to one of the preceding claims, in which the relative position (R) is determined using learning methods.

## Revendications

1. Procédé de déplacement d'un bras (6) porte-instrument d'un robot (4) de laparoscopie dans le cadre d'une préparation d'une opération, le déplacement du bras (6) porte-instrument s'effectuant à l'extérieur d'un patient (2), de manière à ce qu'un instrument (10) mis sur le bras (6) de porte-instrument soit placé dans une position (R) relative, pouvant être donnée à l'avance, par rapport à un trocart (14) placé dans le patient (2), l'instrument (10) étant, dans la position (R) relative, disposé à l'extérieur du patient (2) à une distance de sécurité du trocart (14) et
- on met sur le trocart (14) un marqueur (26a) de position pouvant être mis en position à l'extérieur du patient (2),
- on détecte la position (P) d'emplacement du trocart (14) au moyen du marqueur (26a) d'emplacement,
- on détermine la position (S) de consigne du bras (6) de porte-instrument à partir de la position (P) d'emplacement et de la position (R) relative,
- on détecte la position (I) réelle du bras (6) de porte-instrument,
- au moyen de la position (I) réelle, de la position (S) de consigne et d'un procédé (36) de minimisation d'erreur, on met le bras (6) de porte-instrument dans la position (S) de consigne.

2. Procédé suivant la revendication 1, dans lequel on met le bras (6) de porte-instrument automatiquement dans la position (S) de consigne.

3. Procédé suivant l'une des revendications précédentes, dans lequel on choisit comme position (R) relative une position, qui assure une orientation coaxiale d'un instrument (10) mis sur le bras (6) de porte-instrument et pouvant être introduit dans le trocart (14) par son axe (16) d'instrument le long d'un axe (18) du trocart.

4. Procédé suivant l'une des revendications précédentes, dans lequel on détermine la position (P) d'emplacement et/ou la position (I) réelle et la position (S) de consigne, par une détection optique par un appareil (28) photographique.

5. Procédé suivant la revendication 4, dans lequel on met, comme appareil (28) photographique, un endoscope sur le bras (6) de porte-instrument.

6. Procédé suivant l'une des revendications précédentes, dans lequel on utilise comme bras (6) de porte-instrument un bras de combinaison pour une technique à orifice unique.

7. Procédé suivant l'une des revendications précédentes, dans lequel on effectue, comme procédé (36) de minimisation d'erreur, un procédé visual-servoing.

8. Procédé suivant la revendication 7, dans lequel on effectue, comme procédé (36) de minimisation d'erreur, un procédé visual-servoing à base d'image.

9. Procédé suivant la revendication 8, dans lequel on effectue le procédé (36) de minimisation d'erreur à l'aide d'une matrice (44) jacobienne d'image.

10. Procédé suivant l'une des revendications précédentes, dans lequel on obtient la position (R) relative par un procédé d'apprentissage.
